Europäisches Patentamt

European Patent Office

Office européen des brevets

(19)

(11) Publication number: **0 125 769**

**A2**

(12) # EUROPEAN PATENT APPLICATION

(21) Application number: **84302090.0**

(22) Date of filing: **28.03.84**

(51) Int. Cl.³: **B 01 J 35/04**
**B 01 J 35/08, B 01 J 21/16**
**C 07 C 41/09, C 07 C 43/04**
**C 07 C 85/06**

(30) Priority: **18.04.83 GB 8310409**

(43) Date of publication of application:
**21.11.84 Bulletin 84/47**

(84) Designated Contracting States:
**DE FR GB IT NL**

(71) Applicant: **IMPERIAL CHEMICAL INDUSTRIES PLC**
**Imperial Chemical House Millbank**
**London SW1P 3JF(GB)**

(72) Inventor: **Daniels, James Anthony**
**20a Kingsley Road**
**Frodsham Via Warrington Cheshire WA6 6SG(GB)**

(74) Representative: **Oldroyd, Alan et al,**
**Imperial Chemical Industries PLC Legal Department:**
**Patents Po Box 6**
**Welwyn Garden City Herts, AL7 1HD(GB)**

(54) **Supported catalysts.**

(57) A supported catalyst comprising a catalyst component carried on a support in the form of a rigid inorganic foam made of one or more layer minerals, a process for the preparation of the catalyst by intoducing a catalyst component onto and/or into a pre-formed foam support or by incorporating a catalyst component in the foam support during production of the support and a process for carrying out a chemical reaction by contacting reactants in vapour or liquid form with the supported catalyst. The catalyst affords advantages in terms of one or more of low catalyst loading, reaction efficiency, yield and selectivity.

Croydon Printing Company Ltd.

QM 32680 |EP

## SUPPORTED CATALYSTS

This invention relates to supported catalysts
for use in vapour-phase (gas-phase) and liquid-phase
reactions and in particular to supported catalysts
wherein the support comprises one or more layer
minerals and use of the supported catalyst for carrying
out vapour-phase and liquid-phase reactions.

The layer minerals are well known, naturally-
occurring materials which are used extensively in
industry, for example in the manufacture of ceramics,
refractory materials, insulation materials, papers,
paints, adhesives, plastics and rubbers. They are
naturally-occurring forms of silica and are
phyllosilicate materials, i.e. they have a layer
structure. Included amongst the layer minerals are, for
example, vermiculite, kaolin and kaolinite-containing clays such as China clays, fire clays, and ball clays, montmorillonite, sepiolite, illite, attapuligite and saponite. It has been proposed hitherto to employ some of the layer minerals, particularly clays and montmorillonite in powder form, as catalyst supports.

The present invention resides in the use as catalyst supports of the layer minerals in a particular physical form and in particular in the form of a rigid foam. We have found that use of the layer minerals in the form of a rigid foam affords advantages over use of the layer minerals in other physical forms in terms of one or more of low catalyst loadings, reaction efficiency, yield of desired product(s) and selectivity of the reaction being catalysed.

According to the invention there is provided a supported catalyst comprising a catalyst component carried on a support in the form of a rigid inorganic foam made of one or more layer minerals.

Preferably the rigid inorganic foam is in the form of prills of foam each of cellular structure made of the layer mineral(s) and especially preferred are prills of foam made of ball-clay or another kaolinite-containing clay.

By the term "prills" as used throughout this specification we mean particles, beads, pieces or small lumps of rigid foam having an essentially-continuous cellular structure in which the walls of the cells are constituted by the layer mineral(s), although the term is not intended as implying any particular size, shape or configuration of the pieces of foam. Typically, and as a guide only, the prills will be essentially spherical or cylindrical pieces of foam of maximum dimension below about 5 mm, for example from 0.5 mm to 5 mm.

The production of rigid layer mineral foams is known and foams produced by any of the known processes may be employed as supports in the supported catalysts according to the invention. The production of layer mineral foams is described, for example. in United Kingdom Patent Specification No. 1,585,104 (vermiculite foam) and in United Kingdom Patent Application No. 2,067,174A (layer mineral foams), the disclosures of which are incorporated herein by reference. Prills of layer mineral foams and their production are described in United Kingdom Patent Application No. 2,067,174A in which it is also described that the prills may be assembled, optionally using binders, into products having an essentially-cellular structure.

It is to be understood that products made from prills of foam are included within the term "rigid inorganic foam" as used throughout this specification, even though the cellular structure of such products may not be truly continuous. It is to be understood also that the term "inorganic foam" as applied for example to products comprising prills, does not exclude foams which are essentially inorganic but which may contain a small amount, say up to 20% by weight, of an organic binder used to unite prills of foam into a self-supporting structure.

The preferred catalyst support is a rigid foam comprising a ball-clay or another kaolinite-containing clay, especially such a foam in the form of prills. One of the main reasons for this preference for kaolinite-containing clay foams is that such clays result in hard foams of high compressive strength which tend to resist handling wear and high-pressure conditions better than do foams made from the other layer minerals. Since foams of high strength are desirable, we prefer to employ ball clay foams which have been sintered by heating them at a temperature of up to about 1500°C, say 1000°C. Enhancement of the strength of layer mineral foams by sintering is described in United Kingdom Patent Application No. 2,067,174A. In the case of wholly-vermiculite foams sintering does not produce an appreciable enhancement of the strength of the foam and a compressive-strength improver such as particulate magnesium oxide is usually incorporated, as in described in European Patent Application No. 9310A the disclosure of which is incorporated herein by reference. Enhancement of the strength of vermiculite foam prills by incorporating a compressive-strength improver is described in United Kingdom Patent Application No. 2,067,174A.

Irrespective of the layer mineral, a preferred embodiment of the invention resides in a supported catalyst in which the support is a rigid inorganic foam (prills or extruded foam) wherein the mean cell diameter is below 60 microns, the maximum cell size is 150 microns and at least 90% of the cells have a diameter less than 100 microns. Such foams and their production are described in European Publication No.96489 Al.

The density of the layer mineral foam support may vary within wide limits, for example from 60 kg/m$^3$ to greater than 600 kg/m$^3$. In general increasing the density of the foam results in a decrease in the ease of post-incorporation of the catalyst component in the foam structure and a decrease in the loading of the catalyst component on the support. Also whilst the distribution of the catalyst component is uniform in low-density foams, say below 300 kg/m$^3$, the catalyst

component tends to appear mostly in the external surface regions of denser foams, say above 500 $kg/m^3$. In order that the foam be sufficiently robust to withstand preparation, use and handling of the catalyst, we prefer to employ higher-density foams, for example foams of density above 500 $kg/m^3$. The loading of the catalyst component on such foams tends to be rather low in comparison with most supported catalysts but an advantage of the supported catalysts of the invention is that they are effective catalysts even when the loading of the catalyst component is lower than is usual in supported catalysts. Loadings of from 1% to 50% by weight of the catalyst component are typical; in general catalyst component loadings of from 5% to 20% by weight are satsifactory.

The catalyst component of the supported catalyst may be any of the liquid or solid substances known for use as catalysts in chemical reactions. Included amongst the liquid catalysts are organic and inorganic acids, for example various sulphonic acids, triflic acid, phosphoric acid, nitric acid, chlorosulphonic acid and notably sulphuric acid, and the phosphorus halides, e.g. phosphorus pentachloride. Layer mineral foams are generally inert towards acids and will absorb and retain large amounts of liquid acids, for example from five to nine times their own weight of acid. The absorbed liquids are not readily leached from the foams by gas streams or liquid reaction media and are retained for long periods in gas-phase and liquid-phase reactions using the catalyst. Included amongst the known solid catalyst are metals, for example nickel, cobalt, iron and the platinum group metals, and metal salts.

Solid catalyst components can be introduced onto and/or into pre-formed layer mineral foam supports by any of the known techniques which include (a) applying the catalyst in solution in a suitable solvent followed by removal of the solvent, (b) depositing/condensing a vapour on the support and (c) applying a precursor of the catalyst (e.g. in solution) to the support and forming the catalyst in situ on the support. Alternatively the catalyst component may be incorporated in the foam during formation of the foam, for example by incorporating the catalyst component or a precursor thereof in the suspension of the layer mineral prior to gasification of the suspension, or by mixing the catalyst component or precursor thereof with the gasified suspension prior to shaping and removing volatile liquid from the gasified suspension.

The invention includes a process for carrying out catalysed chemical reactions which comprises contacting one or more reactants in vapour phase or liquid-phase with a supported catalyst comprising a catalyst component carried on a support in the form of a rigid inorganic foam made of one or more layer minerals.

Examples of the catalysed reactions which can be carried out according to the invention are:

(i)     Synthesis of methyl tertiary-butyl ether from isobutene and methanol in the presence of an acid catalyst, notably concentrated sulphuric acid,

(ii)    Synthesis of alkylamines from alcohols and ammonia in the presence of cobalt and/or nickel,

(iii)   Synthesis of iso-octane from isobutene and isobutane at 0-15°C using high isobutene:isobutane ratios (e.g. 12:1) in the presence of sulphuric acid, (iv)    Synthesis of xylenes from toluene and methanol at 400-450°C in the presence of sulphuric acid,

(v)     General chlorosulphonation reactions in the presence of chlorosulphonic acid,

(vi)    Adiponitrile synthesis using phosphoric acid as catalyst,

(vii)   Synthesis of ethylene from ethanol and hydrogen, and

(viii)  Oxidation of ethylene to ethylene oxide using a silver catalyst.

The conditions under which the reactions are carried out may be any of those known for carrying out the reactions using the particular catalyst component. For example production of methyl tertiary-butyl ether from methanol and isobutene may be carried out using a supported catalyst under the conditions described in United States Patent No. 4,219,678 and synthesis of alkylamines from alcohols and ammonia in the presence of hydrogen gas may be carried out under the conditions described in European Patent Application No. 36547 A1.

In the production of methyl tertiary-butyl ether, methanol vapour, diluted with an inert gas such as nitrogen in order to ensure a short contact time between the methanol and the catalyst component and to minimise condensation of methanol on the catlyst, and isobutene are passed over the catalyst at a temperature of from 40°C to 100°C, preferably 50°C to 60°C. The methanol may be fed to the reactor as a vapour or alternatively it may be fed in liquid form and allowed to vapourise in the reactor, especially where the reactor temperature is above the boiling point (ca. 64°C) of methanol, for example from 75°C to 85°C. In the latter case the methanol vapour is diluted with an inert carrier gas in the reactor tube. The mole ratio of methanol to isobutene in the gas stream contacted with the supported catalyst may vary within wide limits, for example from 0.5:1 to 5:1. The reaction is conveniently carried out at ambient (approximately atmospheric) pressure although pressures of from 1 bar to 100 bar are suitable.

High conversions, for example 90% or higher, of alkanols to alkylamines can be achieved using the supported catalysts of the present invention. Primary and secondary alcohols may be reacted and those containing from 1 to 12, especially from 2 to 6, carbon atoms are particularly suitable. Alkylamines containing from 1 to 18 carbon atoms may be produced. The process is particularly useful for the production of ethylamines (mono-, di- and tri-ethylamines) from ethanol and ammonia in a liquid-phase or a gas-phase reaction.

The liquid-phase alkylamines reaction is usually carried out in a stirred pressure vessel heated at 150°C to 250°C. The gas-phase reaction is usually carried out by passing the reactant vapours through a bed of the supported catalyst heated at 120°C to 250°C, preferably from 160°C to 220°C under a pressure of from 1 bar to 25 bars. Conveniently the gas-phase reaction can be carried out under ambient (atmospheric) pressure. The mole ratios of hydrogen:ammonia:alcohol may vary within wide limits but as a guide the range 1:1:1 to 10:5:1 is suitable. Preferably, however, hydrogen is present in excess during the reaction.

Example 1

Essentially-cylindrical ball-clay prills of size 4-5 mm length and density 180kg/m$^3$ (formed by gasifying a suspension of Hymod AT ball clay, prilling using a perforated belt and sintering at 1000°C) were soaked in a solution of N. sulphuric acid for 24 hours at room temperature (approximately 20°C). The prills were then separated by filtration, washed with ( 10 ml) water and acetone (20 ml) and dried under vacuum at 100°C for 6 hours. The dry prills contained 6.6% by weight of sulphuric acid.

The dry prills (1 g) were packed into a glass tube of internal diameter 6 mm and the tube was heated to 50°C in a furnace. A stream of isobutene and methanol vapour (isobutene:methanol=1:2) diluted with nitrogen gas was passed continuously at a total rate of 25 ml/min through the tube over the acid-containing prills for 7 hours. Samples of the reaction products were collected at hourly intervals and analysed by gas chromatography.

The products, of which the proportions remained the same over the period of the experiment, were mainly methyl tertiary-butyl ether (MTBE) and a small amount of di-isobutene. Catalyst selectivity to MTBE was 90-95% and to di-isobutene was 0-5%. Catalyst activity was 0.35-0.4 g MTBE/g catalyst/hour.

For purposes of comparison, MTBE was produced under the conditions described above but using powdered Hymod AT ball clay (non-foamed) as the catalyst support. The powdered clay was soaked in N. sulphuric acid and treated as described above for the clay prills. The dry powder contained 1.8% by weight of sulphuric acid. Catalyst selectivity was the same as for the ball clay prills but catalyst activity was only 0.1 - 0.2 gMTBE/g catalyst/hour.

Example 2

The procedure described in Example 1 was repeated except that the isobutene:methanol ratio in the reactant stream was 1:1.5 instead of 1:2. Catalyst selectivity to MTBE was 80-85% and to di-isobutene was 15-20%. Catalyst activity was 0.25-0.35 g MTBE/g catalyst/hour.

Example 3

The procedure of Example 1 was repeated except that the reaction temperature in the glass tube was 70°C instead of 50°C. Catalyst selectivity to MTBE was 70-80% and to di-isobutene was 20-30%. Catalyst activity was 0.2 - 0.3 g MTBE/g catalyst/hour.

Example 4

The procedure described in Example 2 was repeated using a reaction temperature of 70°C instead of 50°C. Catalyst selectivity was 40-50% to MTBE and 50-60% to di-isobutene. Catalyst activity was 0.1 - 0.15 g MTBE/g catalyst/hour.

Example 5

The procedure of Example 1 was repeated except that the ball clay prills were soaked in concentrated sulphuric acid instead of N. sulphuric acid. The dry prills contained 39.3% by weight of sulphuric acid. Catalyst selectivity was 85-95% to MTBE and 5-15% to di-isobutene. Catalyst activity was 0.25 - 0.4 g MTBE/ g catalyst/hour.

Example 6

The procedure of Example 1 was repeated using Hymod KC ball clay prills of density 130 kg/m$^3$ instead of the Hymod AT prills. The dry prills contained 5.1% by weight of sulphuric acid. Catalyst selectivity was 80-90% to MTBE and 10-20% to di-isobutene and catalyst activity was 0.15 - 0.25 g MTBE/g catalyst/hour.

Example 7

The procedure of Example 1 was repeated except that the Hymod AT foam prills were heated in 5N sulphuric acid at 90°C for 1 hour. The dry prills contained 35.82% by weight of sulphuric acid. In the MTBE synthesis the ratio of isobutene:methanol was 1:1.5. Catalyst selectivity was 75-80% to MTBE and 20-25% to di-isobutene and catalyst activity was 0.25 - 0.4 g MTBE/g catalyst/hour.

Example 8

The procedure described in Example 1 was repeated except that the prills were soaked in 18% triflic acid ($CF_3SO_3H$) instead of sulphuric acid. The dried prills contained 18.3% by weight of triflic acid. Catalyst selectivity was 65-75% to MTBE and 20-30% di-isobutene and catalyst activity was 0.13 - 0.2 g MTBE/g catalyst/hour.

Example 9

The procedure of Example 1 was repeated except that Hymod KC clay foam prills of density $130kg/m^3$ were used and were soaked in 10% diflic acid ($CF_2HSO_3H$). The dried prills contained 24.9% by weight of diflic acid. Catalyst selectivity was 70-80% to MTBE and 20-30% to di-isobutene and catalyst activity was 0.2 - 0.25 g MTBE/g catalyst/hour.

Example 10

The procedure of Example 1 was repeated except that the prills were soaked in concentrated phosphoric acid ($H_3PO_4$) instead of sulphuric acid and the soaked prills were washed with tetrahydrofuran (20 mls) instead of water and acetone. The dried prills contained 70.9% by weight of phosphoric acid. Catalyst selectivity was 75-85% to MTBE and 5-15% to di-isobutene and catalyst activity was 0.02-0.04 g MTBE/ g catalyst/hour.

Example 11

Acid-impregnated Hymod AT dry foam prills were prepared as described in Example 1 but using 20N sulphuric acid. The dried prills contained 32.8% by weight of sulphuric acid. The prills (5 g) were packed into a glass tube of interal diameter 20 mm and a layer of silica chips was created on top of the layer of prills. The tube was heated at 80°C in a furnace and isobutene and nitrogen gas were passed downwardly through the tube at rates of 5 ml/min and 20 ml/min respectively. Liquid methanol, preheated to 50°C, was fed to the top of the packed tube onto the silica chips where it vapourised and the vapour passed through the tube with the isobutene and nitrogen gas. The ratio of isobutene:methanol vapour was approximately 1:2. The products emerging from the tube were collected and analysed at hourly intervals of time by gas chromatography.

Catalyst selectivity was 90-100% to MTBE and 0.5% to di-isobutene and catalyst activity was 0.3-0.4 g MTBE/g catalyst/hour.

Example 12

Hymod KC ball clay foam prills of density 628 $kg/m^3$ were soaked in a solution of 100 g of nickel nitrate $(Ni(NO_3)_2.6H_2O)$ in 100 ml of water for 24 hours at room temperature. The prills were then separated by filtration, washed with a little acetone and dried under vacuum at 100°C for 6 hours. The dried prills were calcined at 450°C in air for 24 hours after which they were found to contain 3.7% by weight of nickel.

The impregnated prills (5 g) were packed in a glass tube (as in Example 1) and heated at 120°C in a furnace and a stream of nitrogen gas and hydrogen gas (ratio 2:1) was passed through the packed tube at a rate of 5 ml/min for 1 hour. The tube was then heated to 200°C for 1 hour and the nitrogen gas was shut-off. The tube was heated at 300°C and hydrogen gas was passed through it at the rate of 50 ml/min for 1 hour.

The flow of hydrogen gas was maintained whilst the tube (containing the activated supported catalyst) was allowed to cool in the furnace to 200°C. At this tube temperature a stream of hydrogen, ammonia and ethanol in the ratio 5:3:1 was passed through the tube at atmospheric pressure and at a flow rate of 0.1 mole/hour.

The products emerging from the tube were sampled at hourly intervals and analysed by gas chromatography. The process was operated for 24 hours.

Conversion of ethyl alcohol to ethylamines was 58% and the products were ethylamine, diethylamine and triethylamine. Catalyst selectivity was 24% to ethylamine, 53% to diethylamine and 22% to triethylamine.

Example 13

The procedure of Example 12 was repeated except that the prills were soaked in cobalt nitrate solution (100 g $CO(NO_3)_2.6H_2O$ in 100 ml water) instead of nickel nitrate solution. The calcined prills contained 3.5% by weight of cobalt.

The conversion of ethyl alcohol to alkylamines was 89% and catalyst selectivity was 24% to ethylamine, 54% to diethylamine and 21% to triethylamine.

Example 14

The procedure of Example 12 was repeated except that in the catalyst preparation the sequence of soaking in nickel nitrate solution, washing, drying and calcining was carried out twice (using the calcined prills from the first step in the second step). The calcined prills after the second treatment contained 7.3% by weight of nickel.

0125769

-14-

The conversion of ethyl alcohol to alkylamines was 75% and catalyst selectivity was 22% to ethylamine, 55% to diethylamine and 22% to triethylamine.

Example 15

The procedure of Example 13 was repeated except that the temperature of the tube during alkylamines synthesis was 180°C instead of 200°C.

The conversion of ethyl alcohol to alkylamines was 73% and catalyst selectivity was 26% to ethylamine, 54% to diethylamine and 20% to triethylamine.

Examples 16-18

The procedure of Example 12 was repeated three times except that the prills used were of the following density and contained the given amount of nickel:-

Example 16    360 kg/m$^3$    10% by weight nickel
Example 17    130 kg/m$^3$    13% by weight nickel
Example 18    108 kg/m$^3$    15% by weight nickel

Conversions of ethyl alcohol to ethylamines and catalyst selectivities were:-

| % Conversion | % Selectivity to | | |
|---|---|---|---|
| | ethylamine | diethylamine | triethylamine |
| Example 16 - 77 | 28 | 53 | 18 |
| Example 17 - 67 | 35 | 49 | 13 |
| Example 18 - 37 | 48 | 46 | 4 |

Example 19

The procedure of Example 13 was repeated except that prills of density 360 kg/m$^3$ were employed. The calcined prills contained 10% by weight of cobalt.

Conversion of ethyl alcohol to ethylamines was 89% and catalyst selectivity was 30% to ethylamine, 53% to diethylamine and 15% to triethylamine.

Example 20

The procedure of Example 13 was repeated except that prills of density 180 $kg/m^3$ were employed. The calcined prills contained 13% by weight of cobalt.

Conversion of ethyl alcohol to ethylamines was 90% and catalyst selectivity was 45% to ethylamine, 47% to diethylamine and 8% to triethylamine.

Example 21

The procedure described in Example 12 was repeated exept that in preparing the supported catalyst the sequence of soaking, washing, drying and calcining was carried out three times. After the third calcination the prills contained 10% by weight of nickel.

Conversion of ethyl alcohol to ethylamines was 62% and catalyst selectivity was 30% to ethylamine, 51% to diethylamine and 17% to triethylamine.

Example 22

The procedure of Example 21 was repeated except that cobalt nitrate solution (as in Example 13) was used instead of nickel nitrate solution.

Conversion of ethyl alcohol to ethylamines was 84% and catalyst selectivity was 26% to ethylamine, 48% to diethylamine and 22% to triethylamine.

General Observation - Examples 1-22

Each of the catalysed reactions described in Examples 1-22 was carried out for a prolonged period, for example 5 to 30 hours and in each experiment it was observed that loss of the catalyst component from the supported catalyst was negligible.

**CLAIMS**

0125769

1.     A supported catalyst comprising a catalyst component carried on a support in the form of a rigid inorganic foam made of one or more layer minerals.

2.     A supported catalyst as claimed in claim 1 wherein the rigid inorganic foam is in the form of prills of foam each of cellular structure made of the layer mineral(s).

3.     A supported catalyst as claimed in claim 1 or claim 2 wherein the rigid inorganic foam is made of a kaolinite-containing clay mineral.

4.     A supported catalyst as claimed in claim 3 wherein the foam has been sintered by heating at a temperature of up to 1500°C.

5.     A supported catalyst as claimed in claim 1 or claim 2 wherein the foam is made of vermiculite and a compressive-strength improver is incorporated in the foam.

6.     A supported catalyst as claimed in claim 5 wherein the compressive-strength improver is particulate magnesium oxide.

7.     A supported catalyst as claimed in any one of the preceding claims wherein the foam is one in which the mean cell diameter is below 60 microns, the maximum cell size is 150 microns and at least 90% of the cells have a diameter less than 100 microns.

8.     A supported catalyst as claimed in any one of the preceding claims wherein the loading of the catalyst component on the support is from 1% to 50% by weight.

9.      A supported catalyst as claimed in any one of the preceding claims wheren the catalyst component is an acid.

10.      A supported catalyst as claimed in any one of claims 1 to 8 wherein the catalyst component is a metal or a metal salt.

11.      A process for the production of a supported catalyst which comprises introducing a catalyst component onto and/or into a pre-formed rigid inorganic foam support.

12.      A process for the production of a supported catalyst which comprises incorporating a catalyst component into a rigid inorganic foam support during formation of the foam.

13.      A process for carrying out a catalysed chemical reaction which comprises contacting one or more reactants with a supported catalyst comprising a catalyst component carried on a support in the form of a rigid inorganic foam made of one or more layer minerals.

14.      A process as claimed in claim 13 for the synthesis of methyl tertiary-butyl ether wherein isobutane and methanol are contacted with an acid catalyst.

15.      A process as claimed in claim 13 for the synthesis of alkylamines wherein an alcohol and ammonia are contacted with a cobalt and/or nickel catalyst.